Europäisches Patentamt

European Patent Office

Office européen des brevets

(19)

(11) Veröffentlichungsnummer: **0 364 583**
**A1**

## (12) EUROPÄISCHE PATENTANMELDUNG
### veröffentlicht nach Art. 158 Abs. 3 EPÜ

(21) Anmeldenummer: 88906222.0

(22) Anmeldetag: 20.04.88

(86) Internationale Anmeldenummer:
PCT/SU88/00090

(87) Internationale Veröffentlichungsnummer:
WO 89/10562 (02.11.89 89/26)

(51) Int. Cl.⁵: **G01N 33/49**

(43) Veröffentlichungstag der Anmeldung:
25.04.90 Patentblatt 90/17

(84) Benannte Vertragsstaaten:
BE CH DE FR GB IT LI LU NL SE

(71) Anmelder: **VSESOJUZNY KARDIOLOGICHESKY NAUCHNY TSENTR AKADEMII MEDITSINSKIKH NAUK SSSR**
**ul. 3 Cherepkovskaya, 15a**
**Moscow, 121552(SU)**

(72) Erfinder: **MARKOSIAN, Ruben Akopovich**
**Leningradskoe shosse, 8/2-345**
**Moscow, 125171(SU)**
Erfinder: **GABBASOV, Zufar Akhnafovich**
**ul. Malaya Bronnaya, 13-17**
**Moscow, 103104(SU)**
Erfinder: **POPOV, Evgeny Georgievich**
**ul. Osennaya, 2-31**
**Moscow, 121609(SU)**
Erfinder: **GAVRILOV, Ilya Jurievich**
**ul. Osennaya, 2-51**
**Moscow, 121609(SU)**
Erfinder: **POZIN, Evgeny Yakovlevich**
**Rublevskoe shosse, 38-2-544**
**Moscow, 121609(SU)**
Erfinder: **PROSHKIN, Sergei Dmitrievich**
**ul. Osennaya, 2-131**
**Moscow, 121609(SU)**

(74) Vertreter: **Nix, Frank Arnold, Dr.**
**Kröckelbergstrasse 15**
**D-6200 Wiesbaden(DE)**

(54) **VERFAHREN ZUR ANALYSE DER AGGREGATION VON THROMBOZYTEN UND EINRICHTUNG ZUR DURCHFÜHRUNG DESSELBEN.**

(57) Beim verfahren zur Analyse der Aggregation von Thrombozyten mißt man die mittlere Intensität und gleichzeitig die durch Fluktuationen der Thrombozytenzahl oder der Anzahl ihrer Aggregate im optischen Kanal (16) hervorgerufene durchschnittliche quadratische Abweichung der Intensität eines das

Blutmuster (12) durchleuchtenden Lichtstromes von der mittleren Intensität und nach diesen Meßergebnissen beurteilt man den mittleren Radius und die Konzentration der Thrombozyten im Blutmuster (12). Die Einrichtung enthält eine Lichtstromquelle (6), einen Lichtempfänger (11), Einheiten (17 und 19) zur Bestimmung der mittleren Intensität bzw. zur Bestimmung der durchschnittlichen quadratischen Abweichung der Intensität vom Intensitätsmittelwert sowie einen Block (20) zur Ermittlung von Parametern der Thrombozytenaggregation.

FIG.2

# VERFAHREN ZUR ANALYSE DER AGGREGATION VON THROMBOZYTEN UND EINRICHTUNG ZUR DURCHFÜHRUNG DESSELBEN

## Technisches Gebiet

Die Erfindung betrifft medizinische Geräte, insbesondere Verfahren zur Analyse der Aggregation von Thrombozyten und Einrichtungen zur Durchführung dieser Verfahren.

## Zugrundeliegender Stand der Technik

Die Blutzellen haben die Eigenschaft, nach Einwirkung einiger physiologischer Agenzien (im folgenden Aggregationsinduktoren genannt) wie ADP, Thrombozyten-Aktivierungsfaktor, Thrombin, Kollagen, Thromboxan $A_2$ u.a. Aggregate zu bilden. Diese Fähigkeit, Aggregate zu bilden, bedingt die Zentralrolle der Thrombozyten in den Auslösemechanismen der Hämostase. Beliebige Störungen der funktionalen Aktivität von Thrombozyten führen zu pathologischen Änderungen. Die Analyse der spontanen oder induzierten Thrombozytenaggregation nach Einwirkung eines Induktors ermoglicht die Diagnose verschiedener pathologischer Zustände, die mit Störungen von Zellenzuständen bei Hämostase im Zusammenhang stehen. Die spontane Aggregation von Thrombozyten oder die Bildung von Aggregaten mit großen Abmessungen bei einer normalisierten Einwirkung eines Aggregationsinduktors bedingt dabei die Tendenz zu Thrombosen, während eine geschwächte oder fehlende Reaktion auf eine derartige Stimulation eine Blutungstendenz verursacht. Die Konzentration von Blutzellen, insbesondere von Thrombozyten, ist auch das wichtigste Merkmal des physiologischen Zustands des Organismus. Eine Verringerung der Konzentration von zirkulierenden Thrombozyten kann z.B. bei schweren Toxikosen, bei disseminierter intravaskulärer Blutgerinnung, bei ausgedehnten Wunden, bei Störungen der Thrombopoese und anderen Erkrankungen erfolgen. Eine Änderung der Konzentration von Thrombozyten bei ihrer Aggregation in vivo ist ein diagnostisches Merkmal für den Zustand des Hämostasesystems im Organismus.

Bekannt sind mehrere Verfahren zur Analyse der Aggregation von Thrombozyten wie z.B. die optische Mikroskopie, die elektronische Mikroskopie, die konduktometrische Teilchenzählung. Diese Verfahren beruhen auf direkter Zählung von Thrombozyten und auf der Bestimmung von Aggregaten nach ihrer Größe in entsprechend vorbereiteten Blutmustern. Die Methoden der Vorbereitung von Blutmustern führen aber zu wesentlichen Fehlern in den erreichten Ergebnissen infolge der Beeinflussung von Blutzellen durch die in die Blutmuster eingeführten Präparate wie Fixier- und Farbstoffe. Außerdem sind diese Verfahren ziemlich arbeitsaufwendig und für die Untersuchung eines so schnell ablaufenden Vorganges wie die Thrombozytenaggregation praktisch ungeeignet.

Dem angemeldeten Verfahren liegt ein fotometrisches Verfahren zur Analyse der Aggregation von Thrombozyten am nächsten, bei dem die Intensität eines Lichtstromes nach seinem Durchtritt durch ein Muster der Thrombozytensuspension gemessen wird. Wegen der in der Suspension anwesenden Thrombozyten ist das genormte und durch Thrombozyten angereicherte Plasma trübe. Die Intensitätsstärke des das Muster durchdringenden Lichts wird zur Bestimmung der Thrombozytenkonzentration benutzt. Bei Bildung von Aggregaten wird die Trübung schwächer, und dies wird für die Registrierung der Aggregation benutzt. Zur Durchführung dieses Verfahrens wird das Blutmuster mit einem Lichtbündel durchleuchtet, wobei man die Intensität des das Muster durchlaufenden Lichtstromes registriert. Eine Änderung der Intensität des das Muster passierenden Lichtstromes bei Aggregation von Thrombozyten liefert ein typisches Aggregationsbild, wobei man nach maximaler Erhöhung der Lichtdurchlässigkeit die Kinetik und den Grad der Thrombozytenaggregation beurteilt (Journal of Physiology, vol. 162, 1962, London, Born B. V.R.Quantitative investigation into the aggregation of blood platelets, p. 67).

Die bekannten Einrichtungen zur Durchführung dieses Verfahrens enthalten eine Lichtquelle, eine Vorrichtung zum Halten und Rühren des Blutmusters und ein fotoempfindliches Element, welches den das Blutmuster durchdringenden Lichtstrom in ein elektrisches Signal umwandelt.

Bekannt ist z.B. ein zur Untersuchung der Aggregation bestimmtes Gerät mit Autorangieren des Ausgangssignals. In diesem Gerät wird als Referenzsignal ein elektrisches Signal benutzt, welches die Lichtdurchlässigkeit eines an Thrombozyten verarmten autologen Plasmamusters angibt. Das Gerät hat zwei Abteilungen mit Küvetten für die Aufnahme eines an Thrombozyten reichen bzw. armen Plasmas (US, A, 4135818; US, A, 3989382).

Es ist auch eine Einrichtung zur Messung der Aggregation von Thrombozyten bekannt, in der die Vorrichtung zum Halten des Musters mit Thrombozyten in der Art eines Hohlzylinders ausgeführt ist, wobei das Muster durch Drehung des Zylinders um seine Achse verrührt wird (US, A, 4066360).

Außerdem ist eine Einrichtung zur Untersuchung von Bluteigenschaften bekannt, in der eine Lichtquelle in der Art einer Lampe, eine Küvettenabteilung, ein magnetisches Rührwerk am Boden der Küvettenabteilung benutzt werden. Nach dem Durchtritt durch das Muster wird der Lichtstrom mit Hilfe eines Lichtempfängers in ein elektrisches Signal umgewandelt, welches der Intensität des hindurchgegangenen Lichtstromes entspricht. Eine derartige Einrichtung kann ohne irgendwelche Änderungen nur für die Analyse der Thrombozytenaggregation angewandt werden (US, A, 4116564).

Ein Mangel dieses bekannten Verfahrens und der Einrichtungen zu seiner Realisierung besteht darin, daß es unmöglich ist, auf Grund von Änderungen der Lichtdurchlässigkeit eines mit Thrombozyten angereicherten Plasmamusters die Größe der entstehenden Aggregate zu bestimmen. Dabei sind auch Fälle möglich, wenn zwei Muster verschieden große Aggregate mit unterschiedlicher Zahl von Thrombozyten haben, aber gleiche optische Dichte aufweisen. Die Form von Thrombozyten beeinflußt die optische Dichte auch wesentlich. Die Abwandlung der Thrombozyten von flachen Scheiben zur Rundform verändert die optische Dichte des Musters um 30 bis 40%. Dies erschwert die differentielle Diagnostik einer erhöhten Thrombozytenaktivität in jedem konkreten Fall. Die technische Realisierung des Verfahrens wird auch infolge der Abhängigkeit seiner Ergebnisse von der Lichtabsorption im Medium (Blutplasma) kompliziert.

Das oben beschriebene Verfahren und die Einrichtungen zu seiner Realisierung weisen eine Empfindlichkeitsschwelle auf. Dies verhindert die Untersuchung der Spontanaggregation von Thrombozyten sowie der Aggregation nach Ein-

wirkung schwacher Aktivatorkonzentrationen, wenn die mittlere Zahl von Thrombozyten in einem Aggregat nicht größer als 100...200 ist. Die niedrige Empfindlichkeit erschwert, in einigen Fällen schließt auch die Möglichkeit aus, die Hyperaktivität der Thrombozyten zu diagnostizieren. In einem realen Blutfluß ist außerdem das Erscheinen von Induktoren gerade in kleinen Konzentrationen möglich, wobei die hemmende Beeinflussung der Aggregation durch einige Arzneimittel nur nach Einwirkung solcher physiologischen Konzentrationen von Induktoren erkannt wird.

Die optische Dichte von Mustern ist nicht nur von der Teilchenkonzentration, sondern auch von optischen Eigenschaften der Teilchen selbst und ihrer Umgebung sowie von der Konstruktion des optischen Kanals der Einrichtung abhängig. Deshalb ist eine ausreichend genaue Bestimmung der Teilchenkonzentration auf Grund der Lichtdurchlässigkeit des Musters mit Teilchen unmöglich.

Offenbarung der Erfindung

Der Erfindung liegt die Aufgabe zugrunde, ein Verfahren zur Analyse der Aggregation von Thrombozyten und eine Einrichtung zur Durchführung desselben zu entwickeln, die es ermöglichen, die Größe der entstehenden Aggregate und mit hoher Genauigkeit die Konzentration von Thrombozyten unabhängig von der Lichtabsorption durch ein Medium und von der Form der Thrombozyten durch Änderung der meßbaren Parameter eines das Muster durchdringenden Lichtstromes zu bestimmen.

Diese Aufgabe wird dadurch gelöst, daß beim Verfahren zur Analyse der Aggregation von Thrombozyten, bei dem ein Blutmuster mit Thrombozyten und/oder ihren Aggregaten mit einem Lichtstrom durchleuchtet wird und die Parameter des Lichtstromes nach seinem Durchtritt durch das Blutmuster gemessen werden, - mittels des das Blutmuster durchdringenden Lichtstromes erfindungsgemäß ein optischer Kanal mit erforderlichen Parametern gebildet wird, im Blutmuster die Bewegung der Thrombozyten und/oder ihrer Aggregate durch den optischen Kanal gewährleistet wird, die mittlere Intensität des das Blutmuster passierenden Lichtstromes und gleichzeitig das Quadratmittel der durch Fluktuationen der Thrombozytenzahl und/oder der Anzahl ihrer Aggregate im optischen Kanal hervorgerufenen Abweichung der Intensität des austretenden Lichtstromes von der mittleren Intensität gemessen werden und auf Grund des gemessenen mittleren Intensitätswertes und der durchschnittlichen quadratischen Intensitätsabweichung des hindurchtretenden Lichtstromes der mittlere Radius der Thrombozyten und/oder ihrer Aggregate und/oder die Thrombozytenkonzentration im Blutmuster ermittelt werden.

Zur Bestimmung des mittleren Radius der Thrombozyten und/oder ihrer Aggregate kann die Größe der relativen Dispersion von Fluktuationen des durch das Blutmuster geleiteten Lichtstromes benutzt werden, die als Quadrat des Verhältnisses der durchschnittlichen quadratischen Abweichung der Intensität zum Intensitäts-Mittelwert gefunden wird.

Für die Bestimmung der Thrombozytenkonzentration ist es zweckmäßig, das Verhältnis des Quadrats der Differenz von Logarithmen der mittleren Intensität des das Blutmuster durchdringenden Lichtstromes und der Lichtstromintensität beim Fehlen von Thrombozyten im Blutmuster zur relativen Dispersion der Lichtstromfluktuationen zu benutzen.

Zur Präzisierung des mittleren Radius von Thrombozyten und/oder ihrer Aggregate erwies es sich als vorteilhaft, die anfängliche Volumenkonzentration von Thrombozyten in den Grenzen von 0,1 bis 1 % einzustellen.

Die Aufgabe der Erfindung wird auch dadurch gelöst, daß die Einrichtung zur Analyse der Aggregation von Thrombozyten mit einer Vorrichtung zum Halten des Blutmusters und mit einer mit dieser verbundenen Vorrichtung zur Bewegung von Thrombozyten und/oder ihren Aggregaten im Blutmuster sowie mit einer Quelle eines auf das Blutmuster gerichteten Lichtstromes und einem mit dieser Quelle optisch gekoppelten Lichtempfänger zur Umwandlung des das Blutmuster durchleuchtenden Lichtstromes in ein elektrisches Signal, welches der Intensität dieses durchleuchtenden Lichtstromes entspricht, - erfindungsgemäß auch mit folgenden Baueinheiten ausgestattet wird: einer mit dem Eingang an den Lichtempfängerausgang angeschlossenen Einheit zur Bestimmung der mittleren Intensität; einer eingangsseitig mit dem Lichtempfängerausgang verbundenen Schaltung zur Unterdrückung von Intensitätsfluktuationen, die durch Drehung von nichtsphärischen Thrombozyten im Strom bedingt sind; einer mit dem Eingang an den Ausgang dieser Schaltung zur Unterdrückung von Fluktuationen angeschlossenen Einheit zur Feststellung der durchschnittlichen quadratischen Abweichung von der mittleren Intensität; einem Block zur Ermittlung der Thrombozytenaggregations-Parameter, die auf den mittleren Radius von Thrombozyten und/oder ihren Aggregaten und/oder auf die Konzentration von Thrombozyten hinweisen, wobei die Eingänge dieses Blocks mit den Ausgängen der Einheit zur Bestimmung der mittleren Intensität und der Einheit zur Bildung der durchschnittlichen quadratischen Intensitätsabweichung verbunden sind.

Es ist zweckmäßig, die Einheit zur Bestimmung der mittleren Intensität mit einem Tiefpaßfilter zu versehen und in der Schaltung zur Unterdrückung von Intensitätsfluktuationen, die durch Drehung von nichtsphärischen Thrombozyten im Strom bedingt sind, ein Hochpaßfilter mit einer Grenzfrequenz von 100 bis 200 Hz anzuwenden.

Die Schaltung zur Unterdrückung von Intensitätsfluktuationen kann ein Bauelement zum Abtrennen der Wechselkomponente des elektrischen Signals enthalten.

Dieses Bauelement zum Herauslösen der Wechselkomponente des elektrischen Signals kann als Hochpaßfilter realisiert werden.

Es erwies sich als vorteilhaft, den Block zur Ermittlung von Parametern der Thrombozytenaggregation, die den mittleren Radius der Thrombozyten und/oder ihrer Aggregate angeben, mit einem Signalteiler zu versehen, dessen Eingänge mit den Ausgängen der Einheit zur Bestimmung der mittleren Intensität und der Einheit zur Feststellung der durchschnittlichen quadratischen Intensitätsabweichung von der mittleren Intensität verbunden werden, und durch eine Quadrierschaltung zu ergänzen, bei welcher der Eingang mit dem Ausgang des Signalteilers verbunden wird und am Ausgang ein dem mittleren Radius von Thrombozyten und/oder ihren Aggregaten proportionales Signal erzeugt wird.

Zweckmäßigerweise kann der Block zur Ermittlung der Thrombozyten-Aggregationsparameter, die auf die Konzentration der Thrombozyten hinweisen, folgende Baueinheiten enthalten: einen an den Ausgang der Einheit zur Bestimmung der mittleren Intensität angeschlossenen Speicherblock; eine erste Logarithmierschaltung, deren Eingang mit dem Ausgang des Speicherblocks verbunden ist; eine zweite Logarithmierschaltung, deren Eingang an den Ausgang der Einheit zur Bestimmung der mittleren Intensität geschaltet ist; eine Subtraktionsschaltung, deren Eingänge an den Ausgängen der ersten und der zweiten Logarithmierschaltung liegen, und einen zweiten Signalteiler, bei dem ein Eingang mit dem Ausgang des ersten Signalteilers verbunden ist, der zweite Eingang am Ausgang der Subtraktionsschaltung liegt und der Ausgang an den Eingang einer zweiten Quadrierschaltung angeschlossen ist, deren Ausgang ein der Thrombozytenkonzentration proportionales Signal liefert.

Kurze Beschreibung der Zeichnungen

Die Erfindung wird in der nachstehenden Beschreibung von Ausführungsbeispielen mit Bezugnahme auf die beigefügten Zeichnungen näher erläutert. Hierbei zeigen

Fig. 1 a,b Zeitabhängigkeitskurven zur Charakteristik von Ergebnissen der Analyse der Thrombozytenaggregation;

Fig. 2 ein Blockschaltbild der Einrichtung zur Analyse der Thrombozytenaggregation gemäß der Erfindung;

Fig. 3 Kurven zur Charakteristik von Änderungen der relativen Dispersion von Lichtstrom-Fluktuationen;

Fig. 4 Schaltungen zur Realisierung einzelner Baueinheiten derselben Einrichtung gemäß der Erfindung;

Fig. 5 eine Schaltung des Blocks zur Ermittlung von Parametern der Thrombozytenaggregation gemäß der Erfindung.

Beste Ausführungsvariante der Erfindung

Das Verfahren zur Analyse der Aggregation von Thrombozyten besteht darin, daß man ein Blutmuster (Blutprobe) mit Thrombozyten und/oder ihren Aggregaten in eine Küvette gibt und mit einem Lichtstrom durchleuchtet, wobei in diesem Blutmuster ein optischer Kanal gebildet wird. Man läßt die Thrombozyten und/oder ihre Aggregate im Blutmuster den optischen Kanal durchlaufen und mißt die mittlere Intensität des austretenden Lichtstromes I, gleichzeitig auch die durchschnittliche quadratische Abweichung $\sigma$ der Intensität des austretenden Lichtstromes von der mittleren Intensität. Die durchschnittliche quadratische Abweichung ergibt sich im Ergebnis von Fluktuationen der Thrombozytenzahl im optischen Kanal. Ausgehend von I und $\sigma$ bestimmt man den mittleren Radius der Thrombozyten und/oder ihrer Aggregate oder die Konzentration der Thrombozyten, oder gleichzeitig den mittleren Radius der Thrombozyten und/oder ihrer Aggregate und die Thrombozytenkonzentration. Für die Bestimmung des mittleren Radius der Thrombozyten und/oder ihrer Aggregate kann man bequem die Größe der relativen Dispersion D der Fluktuationen des das Blutmuster durchdringenden Lichtstromes benutzen, die als Quadrat des Verhältnisses der durchschnittlichen quadratischen Abweichung der Lichtstromintensität zum Mittelwert der Lichtstromintensität, also $D = \left[\dfrac{\sigma}{I}\right]^2$ definiert wird. Für die Ermittlung der Thrombozytenkonzentration benutzt man das Verhältnis des Quadrats der Differenz der Logarithmen der mittleren Intensität I des das Blutmuster passierenden Lichtstromes und der Lichtstromintensität $I_0$ beim Fehlen von Thrombozyten im Blutmuster zur relativen Dispersion D der Lichtstromfluktuationen.

Zur Präzisierung des mittleren Radius der Thrombozyten und/oder ihrer Aggregate in der zur Untersuchung vorbereiteten Thrombozytensuspension bestimmt man die Volumenkonzentration der Thrombozyten durch Zentrifugieren oder mit Hilfe einer Einrichtung, welche die Information über die Volumenverteilung von Teilchen liefert. Durch Verdünnung mit einem Plasma ohne Thrombozyten wird die anfängliche Volumenkonzentration der Thrombozyten in den Grenzen von 0,1 bis zu 1 % eingestellt. In allen Fällen, wenn der Vergleich von Ergebnissen der Bestimmung des mittleren Radius der in verschiedenen Proben entstehenden Aggregate erforderlich ist, hält man sich dabei nur an einen Wert der Volumenkonzentration von Thrombozyten, z.B. an 0,25 %. Die Wahl der Grenzwerte 0,1 und 1 % ist dadurch bedingt, daß bei einer Volumenkonzentration von Thrombozyten unter 0,1 % ihre Konzentration höchstens bei 50 Tausend/µl liegt, wobei die von den optischen Untersuchungsmethoden geforderte Genauigkeit nicht erreicht werden kann. Im Blut von gesunden Menschen ist die Volumenkonzentration der Thrombozyten selten größer als 1,0 %.

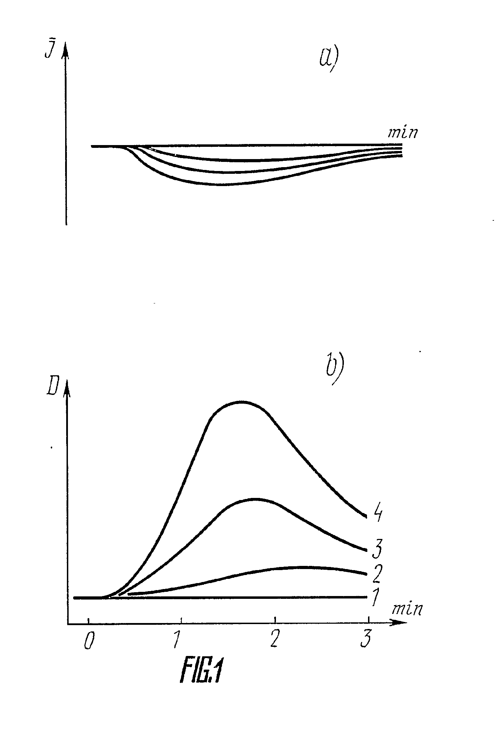

Fig. 1 zeigt die Ergebnisse der Analyse der Thrombozytenaggregation nach Einwirkung verschiedener ADP-Konzentrationen auf die Thrombozyten eines gesunden Menschen. Die Zugabe geringer Konzentrationen von ADP (0,1 µl, 0,15 µl und 0,2 µl) zum Blutmuster bewirkt eine Verringerung der Intensität I (Fig. 1a) des das Blutmuster durchdringenden Lichtstromes, was als Ergebnis der Bildung von Aggregaten nicht interpretiert werden kann, wobei man also die Größe der Thrombozytenaggregate nach dem Intensitätswert nicht eindeutig beurteilen kann. Nach Einwirkung solcher ADP-Konzentrationen erfolgt die dosisabhängige Vergrößerung der Dispersion von Intensitätsfluktuationen des austretenden Lichts, wie dies die Kurven 1, 2, 3, 4 in Fig. 1b zeigen. Das steht mit dem Anstieg der mittleren Größe der Thrombozytenaggregate im Zusammenhang. Aus den Kurven 1...3 ist auch zu ersehen, daß die mittlere Größe der Aggregate nach Zugabe von ADP im Verlaufe von 1 bis 3 min den Maximalwert erreicht und dann kleiner wird. Die Kontrolle mit Hilfe der abtastenden Mikroskopie hat gezeigt, daß im Blutmuster nach Einwirkung von ADP in einer Konzentration von 0,1... 0,2 µl Aggregate entstehen, die auf Grund des Anstiegs von D registriert werden. Dies ermöglicht die Benutzung dieses Parameters (D) zur Durchführung der Analyse der Thrombozytenaggregation.

In Tabelle 1 sind Ergebnisse der Bestimmung der Konzentration von Thrombozyten, Erythrozyten und Latexteilchen mit einer Größe von 4 µm mit Hilfe des betrachteten Verfahrens und der Einrichtung zur Analyse der Aggregation von Thrombozyten aufgeführt.

Tabelle I

| | Thrombozyten | Erythrozyten | Latexteilchen |
|---|---|---|---|
| bekanntes Verfahren | $2 \times 10^5 \,\mu\mathrm{l}^{-1}$ | $1,2 \times 10^4 \,\mu\mathrm{l}^{-1}$ | $8 \cdot 10^3 \,\mu\mathrm{l}^{-1}$ |
| $\dfrac{(\ln I_0 - \ln I)^2}{V \cdot D}$ | $1,9 \times 10^5 \,\mu\mathrm{l}^{-1}$ | $1,23 \times 10^4 \,\mu\mathrm{l}^{-1}$ | $7,8 \times 10^3 \,\mu\mathrm{l}^{-1}$ |

Hier ist V das Volumen des optischen Kanals, welches bei diesem Experiment $5 \,\mu\mathrm{m}^2$ betrug.

Tabelle 2 zeigt die Werte der Extinktion derselben Muster von Thrombozyten, Erythrozyten und Latexteilchen von $4 \,\mu\mathrm{m}$ Größe wie in der Tabelle 1.

Tabelle 2

| Thrombozyten | Erythrozyten | Latexteilchen |
|---|---|---|
| $2,4 \ \mathrm{cm}^{-1}$ | $2,2 \ \mathrm{cm}^{-1}$ | $2,9 \ \mathrm{cm}^{-1}$ |

Unter Extinktion wird hier der Logarithmus des Verhältnisses des einfallenden Lichtstromes zum austretenden Lichtstrom verstanden.

Aus Tabellen 1 und 2 kann man ersehen, daß die Ergebnisse der Messungen nach dem bekannten und dem angemeldeten Verfahren praktisch übereinstimmen, d.h. eine Änderung der optischen Eigenschäften der Teilchen die Genauigkeit der Anzeige bei Anwendung des vorgeschlagenen Verfahrens und der Einrichtung nicht beeinflußt.

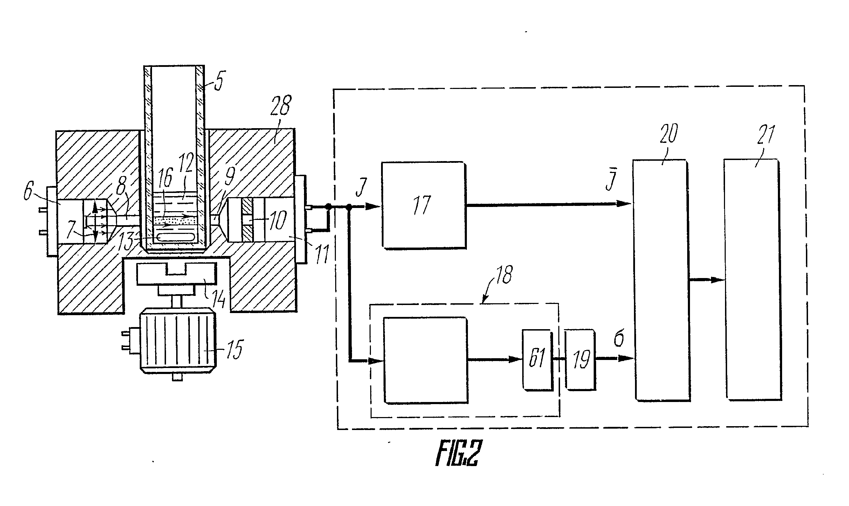

Fig. 2 zeigt ein Blockschaltbild der Einrichtung zur Analyse der Aggregation von Thrombozyten. Die Einrichtung enthält eine Vorrichtung zum Halten eines Blutmusters, die als austauschbare lichtdurchlässige Küvette 5 mit flachem Boden ausgeführt ist. Als Lichtquelle dient ein Halbleiterlaser 6. Der Leuchtkörper des Lasers 6 liegt im Brennpunkt einer kurzbrennweitigen kollimierenden Linse 7. Das zu einem Parallelbündel gesammelte Licht des Lasers 6 passiert eine Eintrittsblende 8 und Austrittsblenden 9 und 10. Das kollimierte Licht der Lichtquelle gelangt nach dem Durchgang durch die Eintrittsblende 8, durch die Küvette 5 und die Austrittsblenden 9 und 10 zu einem Lichtempfänger 11. Der Lichtempfänger 11 wandelt den auf ihn einfallenden Lichtstrom in elektrisches Signal um.

Die Bewegung der Thrombozyten und/oder ihrer Aggregate in dem in der Küvette 5 befindlichen Blutmuster 12 wird mit Hilfe eines magnetischen Rührwerks 13 gewährleistet, welches mittels eines Magneten 14 von einem Elektromotor 15 gedreht wird.

Im Blutmuster 12 bildet der Lichtstrom des Lasers 6 einen optischen Kanal 16, in dem das Rührwerk 13 einen Blutteilchenfluß erzeugt.

Der Ausgang des Lichtempfängers 11 ist mit dem Eingang einer Einheit 17 zur Bestimmung der mittleren Intensität und mit dem Eingang einer Schaltung 18 zur Unterdrückung von Intensitätsfluktuationen verbunden. Der Ausgang der Schaltung 18 liegt am Eingang einer Einheit 19 zur Bestimmung der durchschnittlichen quadratischen Intensitätsabweichung von der mittleren Intensität.

Die Einrichtung enthält einen Block 20 zur Ermittlung von Parametern der Thrombozytenaggregation, an dessen Eingänge die Ausgänge der Einheit 17 und der Schaltung 18 angeschlossen sind und an dessen Ausgang ein Registriergerät 21 geschaltet ist, das auf eine beliebige bekannte Weise z.B. als Sichtanzeigegerät ausgeführt werden kann.

Die Einheit 17 ist als Tiefpaßfilter ausgeführt, während die Schaltung 18 ein Hochpaßfilter mit einer Grenzfrequenz von 100...200 Hz darstellt.

Bei einer Grenzfrequenz unter 100 Hz werden die Intensitätsfluktuationen nicht ausreichend effektiv unterdrückt, und infolgedessen sinkt die Meßgenauigkeit. Bei einer höher als 200 Hz liegenden Grenzfrequenz sinkt die Größe des Nutzsignals.

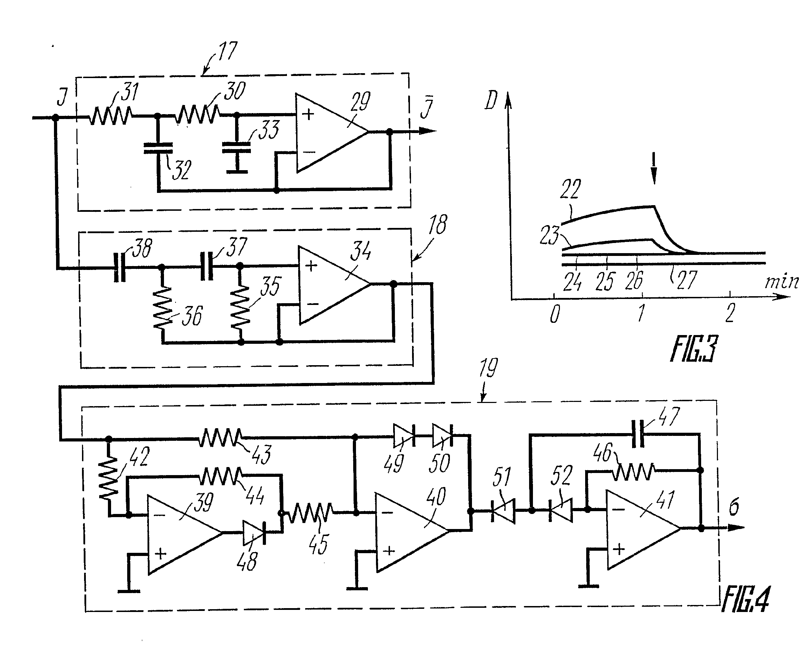

In Fig. 3 sind zeitliche Änderungen der relativen Dispersion von Fluktuationen des das Blutmuster passierenden Lichtstromes bei Benutzung von Hochpaßfiltern mit verschiedenen Grenzfrequenzen: 50 Hz, 100 Hz, 150 Hz, 200 Hz und 400 Hz gezeigt. Untersucht wurde der Einfluß von Änderungen der Thrombozytenform auf die Größe $\left[\dfrac{6}{I}\right]^2 = D$ nach Einwirkung von 0,5 µl ADP auf die Thrombozyten. Infolge der Bindung von Kalziumionen erfolgt in diesem Medium keine Thrombozytenaggregation. In der angemeldeten Einrichtung ohne Filter (Kurve 22) und mit Benutzung eines Filters mit der Grenzfrequenz von 50 Hz (Kurve 23) beeinflußt eine Änderung der Zellenform die Meßergebnisse. Bei Benutzung von Filtern mit den Grenzfrequenzen 100 Hz, 150 Hz, 200 Hz und 400 Hz (Kurven 24, 25, 26 und 27) hat eine Änderung der Thrombozytenform bereits keinen Einfluß auf die Ergebnisse, aber bei der Grenzfrequenz von 400 Hz (Kurve 27) wird das Nutzsignal in der Einrichtung kleiner.

Die Küvette 5 (Fig. 2), der Laser 6, die Linse 7, die Blenden 8...10 und der Lichtempfänger 11 befinden sich auf einem Tragkörper 28, wobei die Blenden 8 und 9 gleichachsige Öffnungen im Tragkörper 28 darstellen.

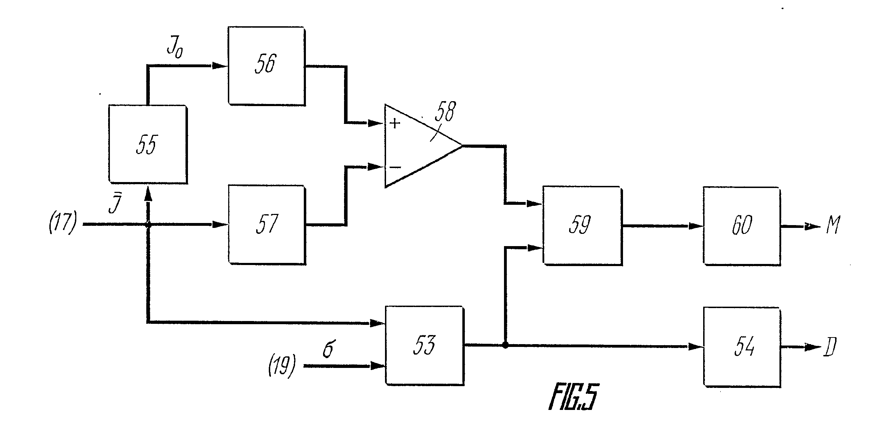

Ein Beispiel der Realisierung der Einheiten 17, 19 und der Schaltung 18 zeigt Fig. 4.

Die Einheit 17 enthält einen Operationsverstärker 29, an dessen Eingang Widerstände 30 und 31 sowie Kondensatoren 32 und 33 liegen.

Zur Schaltung 18 gehören ein Operationsverstärker 34, Widerstände 35 und 36 sowie Kondensatoren 37 und 38.

Die Einheit 19 enthält Operationsverstärker 39, 40, 41, Widerstände 42, 43, 44, 45, einen Kondensator 47 und Dioden 48, 49, 50, 51, 52, die miteinander nach Fig. 4 verbunden sind.

Der zur Ermittlung von Parametern der Thrombozytenaggregation bestimmte Block 20 (Fig. 2) enthält einen Teiler 53 (Fig. 5), dessen Eingänge an die Ausgänge der Einheiten 17 (Fig. 2) und 19 geschaltet sind. Am Ausgang des Teilers 53 (Fig. 5) wird ein dem Ausdruck $\dfrac{6}{I}$ entsprechendes Signal erzeugt, das dem Eingang einer Quadrierschaltung 54 zugeführt wird, deren Ausgang ein der Dispersion D von Fluktuationen des Lichtstromes entsprechendes Signal liefert, der das Blutmuster durchdringt und seinerseits dem mittleren Radius von Thrombozyten und/oder ihren Aggregaten proportional ist.

Zur Bestimmung der Konzentration von Thrombozyten enthält der Block 20 (Fig. 2) einen Speicherblock 55 (Fig. 5), der mit seinem Eingang am Ausgang der Einheit 17 (Fig. 2) liegt und den Wert der mittleren Intensität $I_0$ des Lichtstromes nach seinem Durchgang durch das Blutmuster ohne Thrombozyten speichert. Der Ausgang des Speicherblocks 55 ist mit einer Logarithmierschaltung 56 verbunden, die zusammen mit einer Schaltung 57 zum Logarithmieren der mittleren Intensität I des aus dem zu untersuchenden Blutmuster austretenden Lichtstromes an die Eingänge einer Subtraktionsschaltung 58 angeschlossen ist. Der Ausgang der Schaltung 58 und der Ausgang des Teilers 53 liegen an den Eingängen eines anderen Teilers 59, dessen Ausgang mit dem Eingang einer Quadrierschaltung 60 verbunden ist. Vom Ausgang der Schaltung 60 wird ein Signal M geliefert, welches der Thrombozytenkonzentration im Blutmuster proportional ist.

Nach Fig. 2 enthält die Schaltung 18 ein als Hochpaßfilter realisiertes Bauelement 61 zum Abtrennen der Wechselkomponente des elektrischen Signals. In Fig. 4 sind die beiden Hochpaßfilter zu einem Schaltungsteil vereinigt.

Die erfindungsgemäße Einrichtung wird mit Hilfe von Mikroschaltkreisen und einem Computer realisiert.

Die zur Analyse der Aggregation von Thrombozyten bestimmte Einrichtung funktioniert wie folgt.

Das vorbereitete Blutmuster, das keine Thrombozyten enthält, füllt man in die Küvette 5. Das Licht der Quelle 6 wird von der kurzbrennweitigen kollimierenden Linse 7 zu einem Parallelbündel gesammelt. Die Eintrittsblende 8 formiert aus dem Lichtstrom einen schmalen Strahl, der das Blutmuster 12 in der Küvette 5 durchleuchtet. Nach dem Durchgang durch die Austrittsblenden 9 und 10 gelangt der Lichtstrom weiter zum Lichtempfänger 11. Der Lichtstrom bildet im Blutmuster 12 einen optischen Kanal 16. Das Ausgangssignal des Lichtempfängers 11 wird dem Eingang der Einheit 17 zur Messung der mittleren Intensität und weiter dem Eingang des Speicherblocks 55 zugeführt, in dem es gespeichert und als Signal mit der Information über die Intensität des das Blutmuster durchdringenden Lichtstromes beim Fehlen von Thrombozyten im Muster aufbewahrt wird.

Aus der Küvette 5 wird das Blutmuster ohne Thrombozyten entfernt und statt dessen füllt man in die Küvette 5 ein Blutmuster mit zur Analyse vorbereiteten Thrombozyten. In die Küvette 5 legt man das magnetische Rührwerk 13, und man schaltet den Elektromotor 15 ein. Das Licht der Quelle 6 wird von der kurzbrennweitigen kollimierenden Linse 7 zu einem Parallelbündel gesammelt, und die Eintrittsblende 8 formiert daraus einen schmalen Strahl, der das Blutmuster 12 in der Küvette 5 durchleuchtet. Nach dem Durchgang durch die Austrittsblenden 9 und 10 gelangt der Lichtstrom weiter zum Lichtempfänger 11. Im Blutmuster 12 wird ein optischer Kanal gebildet. Das Ausgangssignal des Lichtempfängers 11 wird den Eingängen der Einheit 17 zur Bestimmung der mittleren Intensität und der Schaltung 18 zur Unterdrückung von Intensitätsfluktuationen zugeführt. Vom Ausgang der Schaltung 18 zur Unterdrückung von Intensitätsfluktuationen wird das Signal auf die Einheit 19 zur Bestimmung der durchschnittlichen quadratischen Abweichung gegeben. Die Bewegung der Thrombozyten und/oder ihrer Aggregate durch den optischen Kanal 16 führt zu Fluktuationen der Zahl von Teilchen in diesem Kanal 16. Dies bewirkt seinerseits das Erscheinen des Signals $\sigma$ am Ausgang der zur Bestimmung der durchschnittlichen quadratischen Abweichung vorgesehenen Einheit 19. Die Ausgangssignale dieser Einheit 19 und der Einheit 17 zur Bestimmung der mittleren Intensität werden dann den Eingängen des Teilers 53 zugeführt. Ein dem Ergebnis der Teilung von $\frac{\sigma}{I}$ entsprechendes Signal gelangt an den Eingang der Quadrierschaltung 54 und wird als D-Signal, das dem mittleren Radius von Thrombozyten und/oder ihren Aggregaten proportional ist, auf das Registriergerät 21 geführt. Das Ausgangssignal der Einheit 17 zur Bestimmung der mittleren Intensität wird auch dem Eingang der Logarithmierschaltung 57 und weiter einem der Eingänge der Subtraktionsschaltung 58 zugeführt. Das im Speicherblock 55 gespeicherte Signal wird von seinem Ausgang an die Logarithmierschaltung 56 und weiter an den Eingang der Subtraktionsschaltung 58 geleitet. Das Ausgangssignal dieser Schaltung 58 wird auf einen Eingang des Teilers 59 gegeben. Dem zweiten Eingang des Teilers 59 wird das Ausgangssignal des ersten Teilers 53 zugeführt. Weiter gelangt das Ausgangssignal des Teilers 59 an die Quadrierschaltung 60 und wird als Signal $M = \dfrac{(\ln I_0 - \ln I)^2}{D}$ , das der Thrombozytenkonzentration proportional ist, an das Registriergerät 21 geleitet.

Nach der Größe der Signale D und M wird der Aggregationsgrad von Thrombozyten bei ihrer Spontanaggregation oder nach Einwirkung von Aggregationsinduktoren quantitativ eingeschätzt. Die bei Anwendung des angemeldeten Verfahrens und der Einrichtung zu seiner Realisierung erhaltenen Daten ermöglichen die Bestimmung eines Zustands von Thrombozyten mit erhöhter oder geschwächter Aktivität. Dies ist wichtig für die Diagnostik von hämatologischen Erkrankungen (Glanzmann- Thrombasthenie, Bernard-Sulie--Syndrom u.a.) sowie von Herz- und Kreislauf-Krankheiten (Herzischämie, Kardiomyopatie, Hypertonie u.a.) und von einer ganzen Reihe anderer Erkrankungen.

Die Anwendung der angemeldeten Erfindung gestattet es, das Risiko von thromboembolischen Komplikationen herabzusetzen und Wege für die medikamentöse Korrektur von Störungen der Thrombozytenaktivität bei Patienten zu finden. Eine breite Anwendung findet die Erfindung beim Screening von neuen Arzneimitteln und bei der Zustandskontrolle des für die Bluttransfusion vorbereiteten Thrombozytenkonzentrats. Die Erfindung ermöglicht eine kontinuierliche Kontrolle des mittleren Radius von Thrombozyten und/oder ihren Aggregaten in dem zu untersuchenden Muster. Dadurch ergibt sich die Möglichkeit, Änderungen der funktionalen Aktivität von Thrombozyten vollständiger und genau zu charakterisieren. Eine hohe Empfindlichkeit des Verfahrens und der Einrichtung zur Aggregationsanalyse und dementsprechend die Möglichkeit der Bestimmung des Aggregationsgrades von Thrombozyten nach Einwirkung geringer Konzentrationen von Induktoren gestatten es, den Zustand erhöhter Aktivität von Thrombozyten bei Hypertonie, bei Anfangsstadien der Dilatations-Kardiomyopatie und bei einer Reihe anderer Herz-
- und Kreislauferkrankungen zu diagnostizieren.

Außerdem ermöglicht die Erfindung eine höchst genaue
(0,1... 1%) Bestimmung der Thrombozytenkonzentration in dem
zu untersuchenden Muster, ohne vorher irgendwelche Messungen durchzuführen und zusätzliche Angaben über das Blutmuster zu erhalten. Die Unabhängigkeit der Anzeige optischer Eigenschaften von Zellen gestattet die Benutzung des
Verfahrens und der Einrichtung zur Bestimmung der Konzentration und zur Analyse der Aggregation von beliebigen anderen Blutzellen (Erythrozyten, Leukozyten) oder von ähnlichen biologischen Geweben. Die Einfachheit, die operative Durchführung der Analyse und der große Informationswert  ermöglichen eine weitgehende Anwendung der angemeldeten Erfindung in der klinischen und Laborpraxis.

Gewerbliche Verwertbarkeit

Die Erfindung kann zur Diagnostik von Herz- und Kreislaufkrankheiten bei Menschen angewandt werden.

PATENTANSPRÜCHE

1. Verfahren zur Analyse der Aggregation von Thrombozyten, bei dem ein Blutmuster (12) mit Thrombozyten und/oder ihren Aggregaten mit einem Lichtstrom durchleuchtet wird und die Parameter des Lichtstromes nach seinem Durchgang durch das Blutmuster (12) gemessen werden, d a d u r c h g e k e n n z e i c h n e t, daß mittels des das Blutmuster durchdringenden Lichtstromes ein optischer Kanal (16) mit erforderlichen Parametern gebildet wird, im Blutmuster (12) die Bewegung der Thrombozyten und/oder ihrer Aggregate durch den optischen Kanal (16) gewährleistet wird, die mittlere Intensität des das Blutmuster (12) passierenden Lichtstromes und gleichzeitig das Quadratmittel der durch Fluktuationen der Thrombozytenzahl und/oder der Anzahl ihrer Aggregate im optischen Kanal (16) hervorgerufenen Abweichung der Intensität des austretenden Lichtstromes von der mittleren Intensität gemessen werden und auf Grund des gemessenen mittleren Intensitätswertes und der durchschnittlichen quadratischen Intensitätsabweichung des hindurchtretenden Lichtstromes der mittlere Radius der Thrombozyten und/oder ihrer Aggregate und/oder die Thrombozytenkonzentration im Blutmuster (12) ermittelt werden.

2. Verfahren nach Anspruch 1, d a d u r c h g e k e n n z e i c h n e t, daß zur Bestimmung des mittleren Radius der Thrombozyten und/oder ihrer Aggregate die Größe der relativen Dispersion der Fluktuationen des durch das Blutmuster (12) geleiteten Lichtstromes benutzt wird, die als Quadrat des Verhältnisses der durchschnittlichen quadratischen Abweichung der Intensität zum Intensitätsmittelwert gefunden wird.

3. Verfahren nach Anspruch 1 oder 2, d a d u r c h g e k e n n z e i c h n e t, daß zur Bestimmung der Thrombozytenkonzentration das Verhältnis des Quadrats der Differenz der Logarithmen der mittleren Intensität des das Blutmuster (12) durchdringenden Lichtstromes und der Lichtstromintensität beim Fehlen von Thrombozyten im Blutmuster (12) zur relativen Dispersion der Lichtstromfluktuationen benutzt wird.

4. Verfanren nach Anspruch 1 oder 2, d a d u r c h
g e k e n n z e i c h n e t, daß zur Präzisierung des mittleren Radius der Thrombozyten und/oder ihren Aggregaten die
anfängliche Volumenkonzentration der Thrombozyten im Blutmuster (12) in den Grenzen von 0,1 bis 1 % eingestellt wird.

5. Einrichtung zur Analyse der Aggregation von Thrombozyten mit einer Vorrichtung (5) zum Halten des Blutmusters (12) und mit einer mit dieser verbundenen Vorrichtung
zur Bewegung von Thrombozyten und/oder ihren Aggregaten
im Blutmuster (12) sowie mit einer Quelle (6) des auf das
Blutmuster (12) gerichteten Lichtstromes und einem mit dieser Quelle (6) optisch gekoppelten Lichtempfänger (11) zur
Umwandlung des das Blutmuster (12) durchleuchtenden Lichtstromes in ein elektrisches Signal, welches der Intensität
dieses durchleuchtenden Lichtstromes entspricht, g e -
k e n n z e i c h n e t d u r c h folgende Baueinheiten:
eine mit dem Eingang an den Ausgang des Lichtempfängers (11)
angeschlossene Einheit (17) zur Bestimmung der mittleren
Intensität, eine einggangsseitig mit dem Ausgang des Lichtempfängers (11) verbundene Schaltung (18) zur Unterdrückung
von Intensitätsfluktuationen, die durch Drehung von nichtsphärischen Thrombozyten im Strom bedingt sind; eine mit
dem Eingang an den Ausgang dieser Schaltung (18) zur Unterdrückung von Fluktuationen angeschlossene Einheit (19)
zur Bestimmung der durchschnittlichen quadratischen Abweichung von der mittleren Intensität; einen Block (20) zur
Ermittlung der Thrombozytenaggregations-Parameter, die auf
den mittleren Radius der Thrombozyten und/oder ihren Aggregaten und/oder auf die Konzentration der Thrombozyten hinweisen, wobei die Eingänge dieses Blocks (20) mit den Ausgängen der Einheit (17) zur Bestimmung der mittleren Intensität und der Einheit (19) zur Bestimmung der durchschnittlichen quadratischen Intensitätsabweichung verbunden
sind.

6. Einrichtung nach Anspruch 5, d a d u r c h g e -
k e n n z e i c h n e t, daß die Einheit (17) zur Bestimmung der mittleren Intensität ein Tiefpaßfilter enthält.

7. Einrichtung nach Anspruch 5 oder 6, d a d u r c h

gekennzeichnet, daß die Schaltung (18) zur Unterdrückung von Intensitätsfluktuationen, die durch Drehung von nichtsphärischen Thrombozyten in der Strömung bedingt sind, ein Hochpaßfilter einschließt.

8. Einrichtung nach Anspruch 7, dadurch gekennzeichnet, daß das Hochpaßfilter ein Filter mit einer Grenzfrequenz von 100 bis 200 Hz darstellt.

9. Einrichtung nach einem beliebigen der Ansprüche 5 bis 8, dadurch gekennzeichnet, daß die Schaltung (18) zur Unterdrückung von Intensitätsfluktuationen ein Bauelement zum Abtrennen der Wechselkomponente des elektrischen Signals enthält.

10. Einrichtung nach Anspruch 9, dadurch gekennzeichnet, daß das Bauelement zum Abtrennen der Wechselkomponente des elektrischen Signals ein Hochpaßfilter darstellt.

11. Einrichtung nach einem beliebigen der Ansprüche 5 bis 10, dadurch gekennzeichnet, daß der Block (20) zur Ermittlung von Parametern der Thrombozytenaggregation, die den mittleren Radius der Thrombozyten und/oder ihrer Aggregate angeben, einen Signalteiler (53) aufweist, dessen Eingänge mit den Ausgängen der Einheit (17) zur Bestimmung der mittleren Intensität und der Einheit (19) zur Bestimmung der durchschnittlichen quadratischen Intensitätsabweichung von der mittleren Intensität verbunden sind, sowie eine Quadrierschaltung (54), bei welcher der Eingang mit dem Ausgang des Signalteilers (53) verbunden ist und am Ausgang ein dem mittleren Radius der Thrombozyten und/oder ihren Aggregaten proportionales Signal erzeugt wird.

12. Einrichtung nach Anspruch 11, dadurch gekennzeichnet, daß der Block (20) zur Ermittlung der Thrombozyten-Aggregationsparameter folgende Baueinheiten enthält: einen an den Ausgang der Einheit (17) zur Bestimmung der mittleren Intensität angeschlossenen Speicherblock (55); eine erste Logarithmierschaltung (56), deren Eingang mit dem Ausgang des Speicherblocks (55) verbunden ist; eine zweite Logarithmierschaltung (57), deren Eingang an den Ausgang der Einheit (17) zur Bestimmung der mittleren Intensität geschaltet ist; eine Schaltung (58) zur Subtraktion von Signalen, deren Eingänge an den Ausgängen der ersten und der zweiten Logarithmierschaltung (56 bzw. 57) liegen, und einen zweiten Signalteiler (59), bei dem ein Eingang mit dem Ausgang des ersten Signalteilers (53) verbunden ist, der zweite Eingang am Ausgang der Substraktionsschaltung (58) liegt und der Ausgang an den Eingang einer zweiten Quadrierschaltung (60) angeschlossen ist, deren Ausgang ein der Thrombozytenkonzentration proportionales Signal liefert.

FIG.1

FIG.2

EP 0 364 583 A1

FIG.3

FIG.4

FIG.5

# INTERNATIONAL SEARCH REPORT

International Application No PCT/SU 88/00090

## I. CLASSIFICATION OF SUBJECT MATTER (if several classification symbols apply, indicate all) *

According to International Patent Classification (IPC) or to both National Classification and IPC

IPC⁴ G01N 33/49

## II. FIELDS SEARCHED

### Minimum Documentation Searched 7

| Classification System | Classification Symbols |
|---|---|
| IPC⁴ | G01N 33/48 ÷ 33/49 |

Documentation Searched other than Minimum Documentation
to the Extent that such Documents are Included in the Fields Searched *

## III. DOCUMENTS CONSIDERED TO BE RELEVANT *

| Category * | Citation of Document, 11 with indication, where appropriate, of the relevant passages 12 | Relevant to Claim No. 13 |
|---|---|---|
| A | US, A, 4657383 (BRIAN J. BELLHOUSE) 14 April 1987 (14.04.87) see the claims & JP, A, 60-113153, 19.06.85 EP, A3, 141632, 04.06.86 US, A, 4682887, 28.07.87 | 1,2 |
| A | US, A, 4352557 (ERNST LEITZ WETZLAR GmbH) 5 October 1982 (05.10.82) see pages 1(c),(d),(e),(f) & DE, A1, 2942466, 30.04.81 DE, A1, 3009260, 08.10.81 | 1 |
| A | US, A, 4577964 (ORTHO DIAGNOSTICS, INC.) 25 March 1986 (25.03.86) see claims 1,2 & EP, B1, 9307, 21.09.83 NO, A, 792876, 07.03.80 JP, A, 55-37998, 17.03.80 AU, B, 524291 ,09.09.82 | 1 |

./.

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 3 November 1988 (03.11.88) | 13 January 1989 (13.01.89) |

| International Searching Authority | Signature of Authorized Officer |
|---|---|
| ISA/SU | |

Form PCT/ISA/210 (second sheet) (January 1985)

| III. DOCUMENTS CONSIDERED TO BE RELEVANT    (CONTINUED FROM THE SECOND SHEET) | | |
|---|---|---|
| Category * | Citation of Document, with indication, where appropriate, of the relevant passages | Relevant to Claim No |
| A | SU, A1, 1345115 (Odessky meditsinsky institut im N.I. Pirogova) 15 October 1987 (15.10.87)   see the claims | 1 |
| A | SU, A1, 1024841 (Institut problem kriobiologii i kriomeditsiny AN Ukrainskoi SSR) 23 June 1983 (23.06.83) | 5-12 |
| A | SU, A1, 1112278 (Institut problem kriobiologii i kriomeditsiny AN Ukrainskoi SSR) 7 September 1984 (07.09.84) | 5-12 |

-------------------

Form PCT/ISA/210 (extra sheet) (January 1985)